# EUROPEAN PATENT APPLICATION

(11) **EP 4 144 782 A1**
(43) Date of publication of application: **08.03.2023**
(21) Application number: 21306191.4
(22) Date of filing: 01.09.2021
(51) Int. Cl.: C08G 69/06, C08G 69/46, C07K 1/04, C40B 50/00, C08G 69/30

(54) **POLYMERIC SOLUBLE SUPPORT, PROCESS FOR THE SYNTHESIS THEREOF AND USES THEREOF**

(71) Applicant: Synhelix, 91058 Evry-Courcouronnes (FR)
(72) Inventor: RANDRIANJATOVO-GBALOU, Irina, 91058 Evry-Courcouronnes (FR); SAID, Ahmed, 91058 Evry-Courcouronnes (FR); KEBE, Seydina Ibrahima, 91058 Evry-Courcouronnes (FR)
(74) Representative: Icosa

(57) **Abstract**

The present invention relates to a process for the synthesis of an organic molecule, comprising the use of a polymeric soluble support. The invention further relates to the polymeric soluble support, and to a process for the synthesis thereof.

## Description

### FIELD OF INVENTION

The present invention relates to polymers comprising allylmalonate moieties and to their use as a soluble support in methods which require or may be carried out using a support, such as for synthesis processes of organic molecules or molecular biology and biochemistry assays (e.g. ligand binding assay).

### BACKGROUND OF INVENTION

The 1984 Nobel Prize in Chemistry was awarded to Robert Bruce Merrifield, for his development of a methodology for chemical protein synthesis on a solid support (Merrifield, 1963. J Am Chem Soc. 85(14):2149-54). This development has revolutionized the field of chemical synthesis - and later, enzymatic synthesis - of biological molecules where the molecule of interest - or an initiator thereof - is bound to and elongated from a functionalized solid support; but also, the field of biochemical assays, such as enzyme or affinity assays. As explained by Merrifield, *"[t]he reason for this approach is that when the growing peptide chain is firmly attached to a completely insoluble solid particle, it is in a convenient form to be filtered and washed free of reagents and by-products".*

Various types of solid supports are commonly used in the art. For example, functionalized magnetic beads are commonly used as carriers of antigens, antibodies, catalyzers, proteins and nucleic acids; and offer the advantage to be easily operable with automated system including electromagnets. Sepharose beads are also commonly used in biochemistry, for binding antibodies and other proteins in various biochemical assays.

Because of their large size, such solid supports allow to perform synthesis and biochemical assays using filtration systems, such as vacuum filtration, for easily filtering and washing or eluting reagents and by-products.

However, such solid supports are not devoid of drawbacks. First, they may be involved in non-specific interactions with some compounds of the reaction mixture, leading to their accumulation on and close to the support, and formation of undesired aggregates in the reaction mixture. Second, solid supports have a steric hindrance which prevents large compounds of the reaction mixture, such as enzymes for example, to reach the support-bound molecules closer to the support.

For example, in the field of enzymatic nucleic acid synthesis, initiator oligonucleotide primers are typically bound to a solid support. However, DNA polymerization is not as efficient using such initiator oligonucleotide primers attached to a support, as compared to free initiator oligonucleotide primers; but most importantly, only 15 to 20 % of the synthetized oligonucleotides are ultimately recovered, because cutting enzymes fail to access the oligonucleotides close to the support to release them. The loss is therefore substantial.

Facing these issues, others have proposed to carry out assays and synthesis reactions in solution, *i.e.,* in absence of any support. This technique addresses the issue of steric hindrance, but to the detriment of an easy filtering and washing, using, *e.g*., filtration systems, such as vacuum filtration. Moreover, forgoing a support is not practicable in any assay and synthesis - which also explains its lack of popularity.

Chemical soluble supports suitable for oligonucleotide synthesis, for instance chemical polymeric supports have also been developed. For instance, WO2014/058755 discloses a support resin suitable for oligonucleotide synthesis, made of a crosslinked styrenic polymer. Said polymer comprises a functionality of 1, which means that only one oligonucleotide may be fixed per resin particle.

It appears therefore that no means currently available is fully satisfactory, and there remains thus a need for alternative means allowing to carry out assays and synthesis in a fast and easy manner while remaining highly efficient.

Here, the Inventors have surprisingly showed that a polymer based on allylmalonate moieties could readily serve as an efficient and reusable soluble support for carrying out assays and synthesis, while overcoming the drawbacks identified in current means and methods. The allylmalonate moieties may be monoallylmalonate moieties or diallylmalonate moieties.

In particular, the soluble support of the invention provides a high modularity in terms of distance between the binding moieties and an easy handling and allows high yields and highly reproducible syntheses. In addition, the soluble support of the invention provides a high binding capacity: it comprises a functionality of at least 2.

While the present application demonstrates the benefits of such polymer based on allylmalonate moieties in an enzymatic nucleic acid synthesis process, it is readily apparent for one skilled in the art that such polymer based on allylmalonate moieties is usable in any assay and synthesis method which would alternatively be carried out using a solid support.

### SUMMARY

This invention thus relates to a process for the synthesis of an organic molecule, comprising the steps of:
(a) Providing a polymer of formula (I): 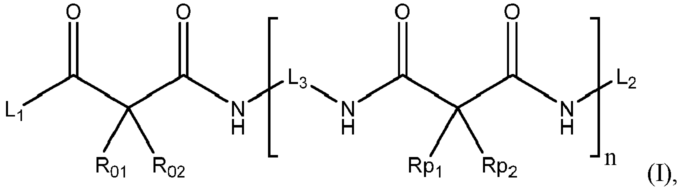 wherein
   L₁ is a terminating group, preferably selected from the group consisting of a OH group, a SH group and a NH₂ group;
   each R_{xy}, with x ranging from 0 to n, and y being 1 or 2, is independently a H atom or a - (CH₂)₂-A_{xy} group, wherein, independently for each x,
      at least one of Rₓ₁ and Rₓ₂ is a -(CH₂)₂-A_{xy} group,
      each A_{xy} is independently a chemical group obtainable by the reaction of an organic compound with a double bond, preferably via a thiol-ene reaction, and in each (Rₓ₁, Rₓ₂) pair, at least one of Aₓ₁ and Aₓ₂ comprises a reactive function suitable for binding of a building block of an organic molecule, preferably a COOH, a NH₂ or an OH group;
   L₂ is a chemical group not comprising any OH, COOH nor NH₂ group, preferably not comprising any reactive function suitable for binding of a building block of an organic molecule;
   n is an integer ranging from 1 to 50, preferably from 2 to 5;
   p is an integer ranging from 1 to n;
   L₃ is a spacer comprising at least 2 consecutive atoms between both nitrogen atoms covalently linked to both extremities of L₃,
      and
(b) Implementing the synthesis of the organic molecule on the polymer of formula (I) provided at step (a) as soluble support.

In one embodiment, the organic molecule is a nucleic acid, a nucleotide, an oligonucleotide, a polynucleotide, an amino acid residue, a peptide, a polypeptide, a protein, a monosaccharide, a disaccharide, an oligosaccharide, a polysaccharide, or a conjugate thereof.

Preferably, the process is an enzymatic nucleic acid synthesis process.

In an embodiment, L₁ is selected from the group consisting of a OH group, a SH group and a NH₂ group, preferably L₁ is a -OH group.

Advantageously, in each (Aₓ₁, Aₓ₂) pair, both Aₓ₁ and Aₓ₂ comprise a reactive function suitable for binding of a building block of an organic molecule, preferably a COOH, a NH₂ or an OH group.

In an embodiment, wherein L₃ comprises 6 consecutive atoms between both nitrogen atoms covalently linked to both extremities of L₃.

Preferably, n is comprised between 2 and 5, preferably n is 2 or 5, more preferably n is 2. In one embodiment, L₂ is a hydrogen atom or an alkyl group.

Advantageously, the soluble support of formula (I) is a polymer of formula (Ia): 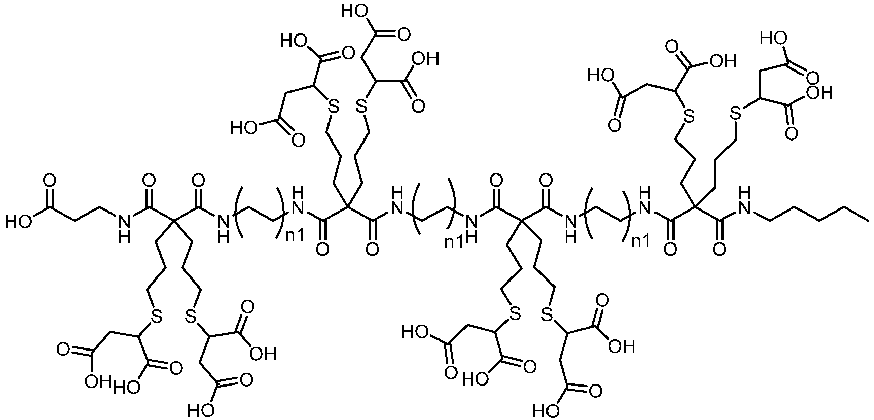 wherein n₁ is an integer ranging from 1 to 9, preferably n₁ is 3.

In one embodiment, the process further comprises at least one step (c) consisting of separating the obtained organic molecule, preferably still bound to the soluble support, from the monomeric building blocks and other small molecular reagents and byproducts. Preferably, the process further comprises at least one step (d) consisting of separating the obtained organic molecule from the soluble support.

The invention also relates to a polymer of formula (I) 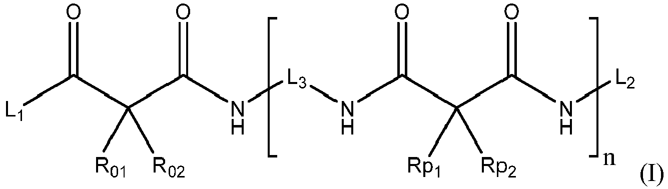 wherein L₁, L₂, L₃, R_{xy}, n and p are as defined above.

The invention also relates to the use of a polymer of formula (I) above, as a soluble support.

The invention also relates to the use of a polymer of formula (I) above, as a soluble support in synthesis process of nucleic acids, preferably a liquid-phase synthesis process of nucleic acids.

Advantageously, the synthesis process of nucleic acids is a *de novo* nucleic acid synthesis process, a template-dependent nucleic acid synthesis process or a nucleic acid assembly process.

The invention also relates to the use of a polymer of formula (I) above, as a soluble support in molecular biology and biochemistry assays.

Preferably, the molecular biology and biochemistry assay is a nucleic acid hybridization assay, a PCR, or a DNA template amplification prior to in vitro transcription.

The invention also relates to a process for the synthesis of a polymer of formula (I) as defined above, wherein the process comprises the steps of:
a1) Providing an intermediate polymer of formula (II) 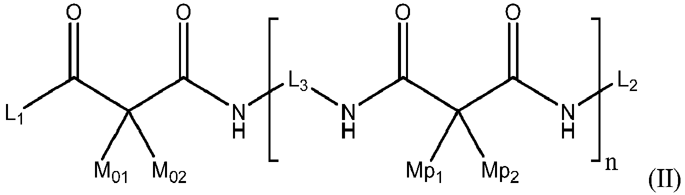 wherein
   each M_{wz}, with w ranging from 0 to n, and z being 1 or 2, is independently a H atom or an allyl -CH₂-CH=CH₂ group; provided that, in each (M_{w1}, M_{w2}) pair, at least one of M_{w1} and M_{w2} is an allyl -CH₂-CH=CH₂ group, preferably in each (M_{w1}, M_{w2}) pair, both M_{w1} and M_{w2} are allyl CH₂-CH=CH₂ groups; and
   L₁, L₂, L₃, n and p are as defined as defined above; and
b1) Reacting the intermediate polymer of formula (II) provided at step (a1) with a compound A, wherein compound A comprises:
   - a reactive function suitable for reacting with a double bond, preferably a reactive function selected from the group consisting of a double bond, a thiol function (SH) and an amine function (NH₂), and
   - a reactive function suitable for fixation of a building block of an organic molecule, preferably a COOH, a NH₂ or an OH group,
      in conditions suitable for implementing a reaction between at least one double bond of the allyl moieties of the intermediate polymer of formula (II) and the reactive function of compound A suitable for reacting with a double bond.

The invention further relates to an intermediate polymer of formula (II): 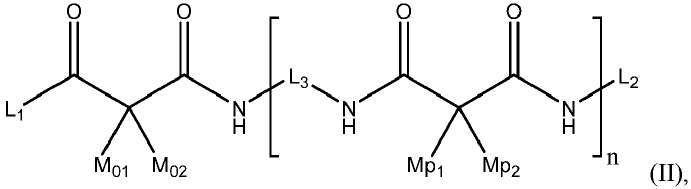 wherein
M_{wz}, L₁, L₂, L₃, n and p are as defined above.

Preferably, the intermediate polymer of formula (II) is selected from the group consisting of: 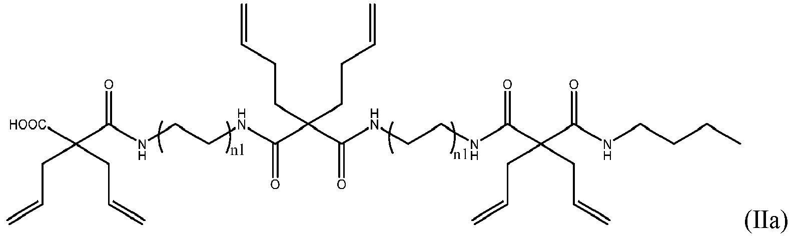 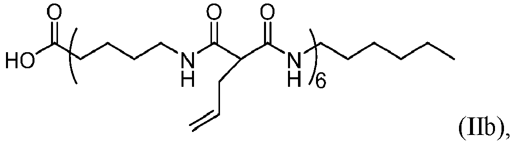 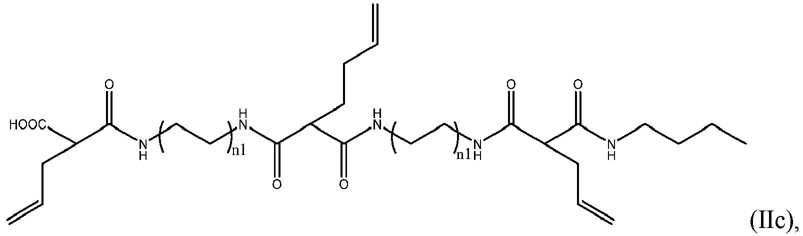 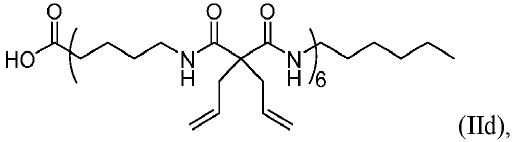 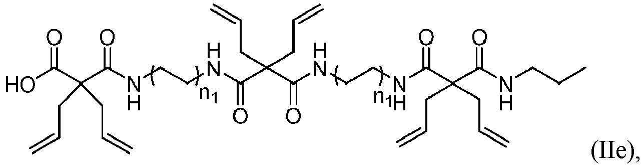 and 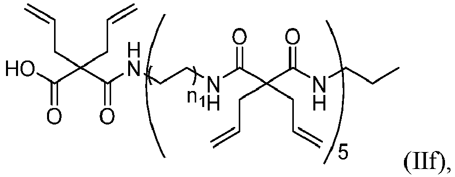 wherein each n₁ is an integer ranging from 1 to 9, preferably n₁ is 3.

### DEFINITIONS

In the present invention, the following terms have the following meanings:
An **"organic molecule"** to be synthesized with a soluble support in the present invention may be any organic molecule which synthesis may be implemented on a soluble support, and preferably which synthesis is allowed and/or improved thanks to the use of a soluble support. Said organic molecule, or a building block thereof, may be modified in order for the organic molecule or the building block thereof to comprise a chemical function suitable for binding to a reactive function of the soluble solid support.

The organic molecule may be a polymeric organic molecule. The term **"polymeric organic molecule"** refers to any organic molecule, which may be formed upon sequential or cyclical coupling of organic molecule building blocks (*e.g.,* in solid-phase syntheses, SPS). The nature of the polymeric organic molecule is dependent upon the identities of the building blocks of said organic molecule. Typically, polymeric organic molecules may include biomolecules, such as, *e.g.,* peptides, polypeptides, oligonucleotides, polynucleotides, oligosaccharides and polysaccharides. Also encompassed are conjugates thereof, such as, *e.g.,* peptide-oligonucleotides conjugates, peptide-polysaccharide conjugates, oligonucleotide-oligosaccharide conjugates, and the like. The polymeric organic molecule may be a biomolecule.

A **"building block"** of an organic molecule is an entity which, when covalently linked to the other building blocks of the same organic molecule, forms said organic molecule. Classically, only one building block among the building blocks of the organic molecule is bound to the soluble support during the synthesis process of the organic molecule (*e.g.,* in solid-phase syntheses). Organic molecule building blocks may be natural or synthetic building blocks, such as amino acids, nucleotides, nucleosides, monosaccharides and derivatives or analogs thereof. The building block of an organic molecule may further include dimers, trimers and the like. In one embodiment, the building block of an organic molecule is selected from the group consisting of an amino acid, a peptide, a nucleotide, a nucleoside, and a saccharide.

In one embodiment, the building block of an organic molecule is protected. The protected building block of an organic molecule may be selected from the group consisting of an *N*-protected amino acid, an *N*-protected peptide, an *O*-protected nucleotide, an *O-*protected nucleoside and an *O*-protected saccharide. Suitable groups for protecting O and/or N atoms of the building blocks are well known in the art and can be routinely selected by one skilled in the art.

A **"soluble support"** is a support suitable for implementing the synthesis of an organic molecule in solution. The synthesis with a soluble support combines the advantageous features of both the solution and solid phase syntheses.

An **"alkyl group"** is a linear, branched or cyclic saturated hydrocarbon moiety comprising from 1 to 24 carbon atoms, preferably from 1 to 12 carbon atoms, in particular from 1 to 6 carbon atoms. Examples of alkyl groups may be methyl group, ethyl group, n-propyl group, iso-propyl group, n-butyl group, tert-butyl group, iso-butyl group, n-pentyl group, iso-pentyl group, tert-pentyl group, n-hexyl group, iso-hexyl group and cyclohexyl group.

An **"aryl group"** is a polyunsaturated, aromatic hydrocarbyl group having a single ring (i.e. phenyl) or multiple aromatic rings fused together (e.g. naphthyl) or linked covalently, typically containing 5 to 12 atoms; preferably 6 to 10, wherein at least one ring is aromatic. A non-limiting example of aryl group is phenyl.

An **"heteroaryl group"** is a 5 to 12 carbon-atom aromatic ring or ring system containing 1 to 2 rings which are fused together or linked covalently, typically containing 5 to 6 atoms on each ring; at least one of which is aromatic and in which one or more carbon atoms in one or more of these rings is replaced by oxygen, nitrogen or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl groups include: triazolyl, pyrrolyl, furanyl, thiophenyl, pyrazolyl, imidazolyl, oxazolyl, isoxazolyl, thiazolyl, isothiazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2-benzisoxazolyl, 2,1- benzisoxazolyl, 1,3-benzothiazolyl, 1 ,2-benzoisothiazolyl, 2,1-benzoisothiazolyl, benzotriazolyl, 1,2,3-benzoxadiazolyl, 2,1 ,3-benzoxadiazolyl, 1,2,3-benzothiadiazolyl, 2,1,3-benzothiadiazolyl, thienopyridinyl, purinyl, imidazo[1,2-a]pyridinyl, 6-oxo-pyridazin-1(6H)-yl, 2-oxopyridin-1 (2H)-yl, 6-oxo-pyrudazin-1(6H)-yl, 2-oxopyridin-1(2H)-yl, 1,3- benzodioxolyl, quinolinyl, isoquinolinyl, cinnolinyl, quinazolinyl, quinoxalinyl. A preferred heteroaryl group used herein is triazolyl.

The term **"heteroatom"** means one or more of oxygen, sulfur, nitrogen, phosphorus, selenium, or silicon (including any oxidized form of nitrogen, sulfur, phosphorus, selenium, or silicon; the quaternized form of any basic nitrogen or; a substitutable nitrogen of a heterocyclic ring, for example N (as in 3,4-dihydro-2H-pyrrolyl), NH (as in pyrrolidinyl) or NR⁺ (as in N-substituted pyrrolidinyl)).

The term **"unsaturated",** as used herein, means that a moiety has one or more units of unsaturation.

The term **"halogen"** means F, Cl, Br, or I.

As used herein, the term **"biomolecule"** refers to any organic molecule that is part of, or from, a living organism. Examples of biomolecules include, but are not limited to, a nucleotide, an oligonucleotide, a polynucleotide, an amino acid residue, a peptide, a polypeptide, a protein, a monosaccharide, a disaccharide, an oligosaccharide, a polysaccharide, and the like. In one embodiment, the biomolecule is a nucleic acid or a fragment thereof, such as, *e.g.,* a nucleotide, an oligonucleotide or polynucleotide.

In one embodiment, the biomolecule is a nucleotide. As used herein, the term "nucleotide" refers to molecules comprising a nucleoside and from one to three phosphate groups. In one embodiment, "nucleotide" refers to molecules comprising a nucleoside and three phosphate groups, also named nucleoside triphosphate. "Nucleotides" are the building blocks of DNA and RNA. Examples of nucleotides include, but are not limited to, ribonucleotides (such as adenosine mono-, di- or triphosphate [AMP, ADP, ATP]; guanosine mono-, di- or tri-phosphate [GMP, GDP, GTP]; uridine mono-, di- or tri-phosphate [UMP, UDP, UTP]; and cytidine mono-, di- or triphosphate [CMP, CDP, CTP]); deoxyribonucleotides (such as deoxyadenosine mono-, di- or tri-phosphate [dAMP, dADP, dATP]; deoxyguanosine mono-, di- or tri-phosphate [dGMP, dGDP, dGTP]; thymidine mono-, di- or tri-phosphate [dTMP, dTDP, dTTP]; and deoxycytidine mono-, di- or tri-phosphate [dCMP, dCDP, dCTP]); and cyclic nucleotides (such as cyclic adenosine monophosphate [cAMP], cyclic guanosine monophosphate [cGMP], cyclic di-guanosine monophosphate [c-di-GMP], cyclic diadenosine monophosphate [c-di-AMP], and cyclic adenosine diphosphate ribose [cADPR]).

In one embodiment, the biomolecule is an oligonucleotide. As used herein, the term **"oligonucleotide"** refers to a short-length, single-stranded, polymer of nucleotide monomers. Oligonucleotides typically comprise from two to about one hundred nucleotide monomers. The term refers to all combinations of nucleotides as defined above, forming a polymer of nucleotides, such as, *e.g.,* a combination of deoxyribonucleotides forming a DNA oligonucleotide, a combination of ribonucleotides forming an RNA oligonucleotide, or a combination of both deoxyribonucleotides and ribonucleotides forming a mixed DNA/RNA oligonucleotide.

In one embodiment, the biomolecule is a polynucleotide. As used herein, the term **"polynucleotide"** refers to a long-length, single-stranded, polymer of nucleotide monomers. Polynucleotides typically comprise more than about one hundred nucleotide monomers. The term refers to all combinations of nucleotides as defined above, forming a polymer of nucleotides, such as, *e.g.,* a combination of deoxyribonucleotides forming a DNA polynucleotide, a combination of ribonucleotides forming an RNA polynucleotide, or a combination of both deoxyribonucleotides and ribonucleotides forming a mixed DNA/RNA polynucleotide.

As used herein, the terms **"linker"** or **"spacer"** are used interchangeably and refer to a chemical moiety that can connect two other moieties (such as, *e.g.,* a polymer moiety or a biomolecule) either covalently, or noncovalently, *e.g*., through ionic or hydrogen bonds or van der Waals interactions.

In one embodiment, the linker or spacer has a backbone of 50 atoms or less, preferably of 40 atoms or less, more preferably of 30 atoms or less, even more preferably of 20 atoms or less.

In one embodiment, the linker or spacer is a covalent bond that connects two moieties. In one embodiment, the linker or spacer is a chain comprising between 1 and 20 atoms in length, for example a chain of about 1, 2, 3, 4, 5, 6, 7, 8, 9, 10, 11, 12, 13, 14, 15, 16, 17, 18, 19 or 20 atoms in length.

In one embodiment, the linker or spacer may be linear, branched, cyclic or a single atom.

In one embodiment, the linker or spacer backbone may comprise or consist of carbon atoms; or alternatively may comprise or consist of a combination of carbon atoms and heteroatoms; or alternatively may comprise or consist of heteroatoms.

In one embodiment, the linker or spacer backbone may comprise or consist of an acyl, acylamino, acyloxy, alkoxy, alkoxycarbonyl, alkoxycarbonylamino, alkyl, trihaloalkyl, alkenyl, alkynyl, amino, amido, imino, aminocarbonyl, aminocarbonylamino, aminocarbonyloxy, aryl, aryloxy, azido, diazo, carboxyl, carbonyl, cyano, isocyanate, isothiocyanate, cycloalkyl, guanidyl, halogen, heterocyclyl, heterocyclyloxy, hydroxyl, keto, nitro, nitroso, oxo, thio, thioether, thioalkoxy, thioaryloxy, thioketo, thiol, sulfonate, sulfinate, phosphinate, phosphonate, alkyl-S(O)-, aryl-S(O)-, alkyl-S(O)₂- and/or arylS(O)₂ group.

In one embodiment, the bonds between backbone atoms of the linker or spacer may be saturated or unsaturated. In one embodiment, the linker or spacer may comprise up to three unsaturated bonds, such as 1, 2 or 3.

For clarity, the number of atoms in the backbone of the linker or spacer is calculated by counting the minimum number of covalently linked atoms between the two moieties connected by the linker or spacer, excluding atoms of the two connected moieties themselves. When the linker or spacer includes a cyclic moiety, the shortest path around the ring of the cyclic moiety is counted so that a minimum possible number of atoms that connect the two moieties is calculated.

In one embodiment, the linker or spacer may include one or more substituent groups, such as, *e.g.,* an alkyl, aryl or alkenyl group.

In one embodiment, the linker or spacer may include, without limitations, oligo(ethylene glycol), esters, ethers, thioethers, tertiary amines, and/or alkyls. The alkyls may be linear or branched, such as, *e.g.,* methyl, ethyl, *n*-propyl, 1-methylethyl (iso-propyl), *n*-butyl, *n-*pentyl, 1,1-dimethylethyl (*t*-butyl), and the like.

In one embodiment, the linker comprises or consists in an ester function.

In one embodiment, the backbone of the linker or spacer may include a cyclic group, such as, *e.g.,* an aryl, a heterocycle or a cycloalkyl group, where 2 or more atoms of the cyclic group are included in the backbone.

In one embodiment, the linker or spacer is cleavable. In one embodiment, the linker or spacer is non-cleavable. As used herein, the term "cleavable" with reference to a linker or spacer implies that said linker or spacer can be selectively cleaved to produce two products. Application of suitable cleavage conditions to a molecule containing a cleavable linker or spacer that is cleaved by said cleavage conditions will produce two "cleavage products". Preferably, a cleavable linker or spacer is stable to physiological conditions, until it is contacted with a reagent capable of cleaving the cleavable linker or spacer.

In one embodiment, the polymer of formula (I) is bound to a solid support. In one embodiment, the polymer of formula (I) is covalently bound to a solid support.

Exemplary materials that can be used as solid supports include, but are not limited to, acrylics, carbon *(e.g.,* graphite, carbon-fiber), cellulose (*e.g.,* cellulose acetate), ceramics, controlled-pore glass, cross-linked polysaccharides (e.g., agarose, SEPHAROSE^{™} or alginate), gels, glass *(e.g.,* modified or functionalized glass), gold *(e.g.,* atomically smooth Au(111)), graphite, inorganic glasses, inorganic polymers, latex, metal oxides (*e.g*., SiO₂, TiO₂, stainless steel), metalloids, metals (e.g., atomically smooth Au(111)), mica, molybdenum sulfides, nanomaterials (e.g., highly oriented pyrolitic graphite (HOPG) nanosheets), nitrocellulose, NYLON^{™}, optical fiber bundles, organic polymers, paper, plastics, polacryloylmorpholide, poly(4-methylbutene), polyethylene terephthalate, poly(vinyl butyrate), polybutylene, polydimethylsiloxane (PDMS), polyethylene, polyformaldehyde, polymethacrylate, polypropylene, polysaccharides, polystyrene, polyurethanes, polyvinylidene difluoride (PVDF), quartz, rayon, resins, rubbers, semiconductor material, silica, silicon (e.g., surface-oxidized silicon), sulfide, and TEFLON^{™}.

In one embodiment, the solid support is a resin, optionally chemically modified. The resin may be a resin based on cross-linked styrene. The resin based on cross-linked styrene may be a Wang resin or a HMPB ChemMatrix^{®} resin. The resin chemically modified may be Fmoc-Gly-Wang resin or Fmoc-Ala-Wang resin.

In one embodiment, the solid support may comprise a magnetic core, such as a ferrimagnetic or superparamagnetic core.

Techniques to covalently attach a peptide, such as a polymer, onto a solid support are well known to the skilled artisan.

The term **"solid-phase synthesis"** or **"SPS",** as used herein, refers to one or a series of chemical or enzymatic reactions carried on to prepare either a single organic molecule, a library of molecularly identical organic molecules or a library of molecularly diverse organic molecules, wherein the chemical or enzymatic reactions are sequentially or cyclically performed on a support. SPS is regularly implemented in the production of organic molecules such as biomolecules, including peptides, nucleic acids molecules, carbohydrates, and conjugates thereof; but could also be applied for synthesis of any organic molecule capable of being synthesized on a solid support.

Solid-phase synthesis processes typically involve the following steps - although one or more of these steps can be modified or adapted depending on the organic molecule to be synthesized, as will readily be apparent to the one skilled in the art:
(a) providing a functionalized support, optionally functionalized with a biomolecule serving as a synthesis initiator;
(b) contacting said functionalized support with at least one organic molecule building block comprising a protecting group, in conditions suitable to couple the organic molecule building block with the functionalized support, optionally with the biomolecule serving as a synthesis initiator, thereby obtaining a protected coupling product;
(c) washing the excess of said protected organic molecule building block;
(d) contacting said functionalized support with at least one deprotecting reagent in conditions suitable to remove the protecting group from the protected coupling product, thereby completing a cycle of solid-phase synthesis of a polymeric organic molecule;
(e) repeating steps (b) to (d) until the desired polymeric organic molecule is obtained.

Examples of synthesis processes of organic molecules in which the soluble support according to the present invention is useful include, but are not limited to, liquid-phase synthesis.

The term **"liquid-phase synthesis"** refers to one or a series of chemical or enzymatic reactions carried out to prepare either a single organic molecule, a library of molecularly identical organic molecules or a library of molecularly diverse organic molecules, wherein the chemical or enzymatic reactions are sequentially or cyclically performed in solution, on a soluble support. The central feature of liquid-phase synthesis is that it combines the advantages that support-less synthesis offers with those that solid-phase synthesis can provide, through the use of a soluble support such as the polymeric scaffold of the invention. Liquid-phase synthesis can be implemented for the production of organic molecules such as biomolecules, including peptides, nucleic acids molecules (including DNA, RNA and mixes thereof), carbohydrates, and conjugates thereof; but could also be applied for synthesis of any organic molecule which would otherwise be synthesized in solution or on a solid support.

In one embodiment, liquid-phase synthesis processes include *de novo* (or template-independent) synthesis, template-dependent synthesis and nucleic acid assembly.

As used herein, the term ***"de novo* synthesis"** refers to a process of synthesizing an organic molecule of any length by iteratively linking organic molecule building blocks until the desired polymeric organic molecule is obtained. *De novo* synthesis processes apply to any type of polymeric organic molecules, including peptides, nucleic acids molecules, carbohydrates, and conjugates thereof. In *de novo* synthesis, the sequence of the newly synthesized organic molecule is not dictated by any template. In the case of *de novo* synthesis (i.e., without template), the process typically involves the following steps - although one or more of these steps can be modified or adapted depending on the organic molecule to be synthesized, as will readily be apparent to the one skilled in the art:
(a) providing a functionalized support, optionally functionalized with a biomolecule serving as a synthesis initiator;
(b) contacting said functionalized support with at least one organic molecule building block comprising a protecting group, in conditions suitable to couple the organic molecule building block with the functionalized support, optionally with the biomolecule serving as a synthesis initiator, thereby obtaining a protected coupling product;
(c) washing the excess of said protected organic molecule building block;
(d) contacting said functionalized support with at least one deprotecting reagent in conditions suitable to remove the protecting group from the protected coupling product, thereby completing a cycle of liquid-phase synthesis of a polymeric organic molecule;
(e) repeating steps (b) to (d) until the desired polymeric organic molecule is obtained.

As used herein, the term **"template-dependent synthesis"** applies typically to the synthesis of nucleic acids, and refers to a process that involves the synthesis of a nucleic acid strand that is complementary to a template strand of interest. In template-dependent synthesis, the sequence of the newly synthesized nucleic acid strand is dictated by complementary base-pairing with the template strand of interest. In the case of template-dependent synthesis, and in particular in the field of nucleic acid synthesis, the process typically involves the following steps - although one or more of these steps can be modified or adapted as will readily be apparent to the one skilled in the art:
(a) providing a functionalized support, optionally functionalized with a nucleotide, oligonucleotide or polynucleotide serving either as:
   - a primer for synthesis of a nucleic acid strand which is complementary to at least a portion of a free nucleic acid template hybridized to said primer; or
   - a nucleic acid template for synthesis of its free complementary nucleic acid strand;
(b) contacting said functionalized support with at least one organic molecule building block, in particular nucleotides or nucleosides, in conditions suitable to either:
   - couple the organic molecule building blocks with the nucleotide, oligonucleotide or polynucleotide serving as a primer in a complementary fashion to the free nucleic acid template; or
   - couple the organic molecule building blocks to one another in a complementary fashion to the nucleotide, oligonucleotide or polynucleotide serving as a nucleic acid template;
(c) repeating step (b) until the desired nucleic acid molecule is obtained.

Typical examples of template-dependent synthesis include enzymatic amplification processes, such as polymerase chain reaction (PCR) which allows to synthetize a DNA (using, e.g., a DNA or RNA-dependent DNA polymerase) or RNA (using, e.g., a DNA or RNA-dependent RNA polymerase) strand from a nucleic acid template (such as, a DNA template or an RNA template). As an exemplary embodiment, methods of template-dependent RNA synthesis are described in Example 4.

As used herein, the term **"nucleic acid assembly"** applies typically to the synthesis of nucleic acids, and refers to a process of assembling a plurality (i.e., two or more) small nucleic acid fragments to produce a longer nucleic acid fragment, using extension-based multiplex assembly reactions, ligation-based multiplex assembly reactions, or a combination thereof, in the presence of a ligase.

In one embodiment, the liquid-phase synthesis processes described above may further include a step of post-synthetic labelling of the polymeric organic molecule, using a modified organic molecule building block. Such modified organic molecule building blocks comprise, e.g., building blocks bearing a fluorescent dye.

In one embodiment, the polymer according to the present invention may be used as a soluble support in molecular biology and biochemistry assays.

**"Molecular biology and biochemistry assays"** may be ligand binding assay, a PCR, or a DNA template amplification prior to in vitro transcription. In one embodiment, "molecular biology and biochemistry assays" is ligand binding assay.

As used herein, the term **"ligand binding assay"** refers to a biochemical test relying on the binding of ligands to a target molecule, such as a receptor, an antibody or any other macromolecule, *e.g*., for screening, detecting and/or quantifying a ligand molecule binding to a given target molecule. In the context of the present invention, the polymer of formula (I) is functionalized with said target molecule.

Examples of ligand/target molecule pairs include, but are not limited to, substrate/enzyme; enzyme/substrate; antigen/antibody; antibody/antigen; polysaccharide/lectin; lectin/polysaccharide; nucleic acid/complementary base sequence; hormone/receptor; receptor/hormone; poly(A) nucleic acid sequence/RNA comprising a poly(U) nucleic acid sequence; metal ion/poly fusion protein; and poly fusion protein/metal ion.

A ligand binding assay may be selected from the group consisting of immunoassay, enzyme assay, nucleic acid hybridization assay and affinity purification.

As used herein, the term **"immunoassay"** refers to a specific type of ligand binding assay in which the presence and/or concentration of a molecule in a solution is measured, through the use of an antibody or an antigen as target molecule. In the context of the present invention, the polymer of formula **(I)** is functionalized with said antibody or antigen. The molecule detected by the immunoassay is referred to as an **"analyte",** and may be, without limitation, a protein or any other type of molecule which may be recognized by or bound by the antibody or antigen.

Examples of immunoassays include, but are not limited to, enzyme-linked immunosorbent assay (ELISA), enzyme-linked immune absorbent spot (ELISpot), radioallergosorbent test (RAST), radioimmunoassay, radiobinding assay, and immunofluorescence assays.

As used herein, the term **"enzyme assay"** refers to a specific type of ligand binding assay in which the activity of an enzyme is measured, *e.g*., for studying enzyme kinetics and/or enzyme inhibition. Typically, the consumption of a substrate or the making of a product by said enzyme, acting as target molecule, is measured. In the context of the present invention, the polymer of formula (I) is functionalized with said enzyme.

As used herein, the term **"nucleic acid hybridization assay",** or in short **"hybridization assay",** refers to a specific type of ligand binding assay in which the annealing of two complementary strands of nucleic acids is detected. In the context of the present invention, the polymer of formula (I) is functionalized with an oligonucleotide, of known or unknown sequence.

As used herein, the term **"affinity purification"** refers to a variant of a ligand binding assay in which a biochemical mixture is separated, based on a specific interaction between a ligand and its target molecule.

### DETAILED DESCRIPTION

### Use as a soluble support

The present invention relates to the use of a polymer of formula (I) 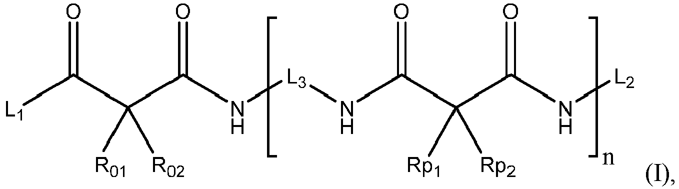 wherein
L₁ is a terminating group, preferably selected from the group consisting of a OH group, a SH group and a NH₂ group;
each R_{xy}, with x ranging from 0 to n, and y being 1 or 2, is independently a H atom or a - (CH₂)₂-A_{xy} group, wherein, independently for each x,
   at least one of Rₓ₁ and Rₓ₂ is a -(CH₂)₂-A_{xy} group,
   each A_{xy} is independently a chemical group obtainable by the reaction of an organic compound with a double bond, preferably via a thiol-ene reaction, and in each (Rₓ₁, Rₓ₂) pair, at least one of Aₓ₁ and Aₓ₂ comprises a reactive function suitable for binding of a building block of an organic molecule, preferably a COOH, a NH₂ or an OH group;
L₂ is a chemical group not comprising any OH, COOH nor NH₂ group, preferably not comprising any reactive function suitable for binding of a building block of an organic molecule;
n is an integer ranging from 1 to 50, preferably from 2 to 5;
p is an integer ranging from 1 to n;
L₃ is a spacer comprising at least 2, preferably from 2 to 10, more preferably 6 consecutive atoms between both nitrogen atoms covalently linked to both extremities of L₃,
as a soluble support.

In an embodiment, the polymer of formula (I) is used as a soluble support in a synthesis process of an organic molecule.

In another embodiment, the polymer of formula (I) is used as a soluble support in molecular biology and/or biochemistry assays.

### Use in a synthesis process of an organic molecule

This invention also relates to the use of a polymer of formula (I) as a soluble support in a synthesis process of an organic molecule.

This invention relates in particular to a process for the synthesis of an organic molecule comprising the steps of:
(a) providing a polymer of formula (I): 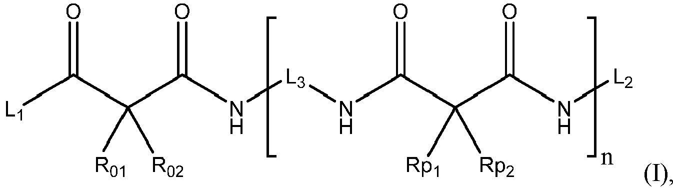 wherein
   L₁ is a terminating group, preferably selected from the group consisting of a OH group, a SH group and a NH₂ group;
   each R_{xy}, with x ranging from 0 to n, and y being 1 or 2, is independently a H atom or a - (CH₂)₂-A_{xy} group, wherein, independently for each x,
      at least one of Rₓ₁ and Rₓ₂ is a -(CH₂)₂-A_{xy} group,
      each A_{xy} is independently a chemical group obtainable by the reaction of an organic compound with a double bond, preferably via a thiol-ene reaction, and in each (Rₓ₁, Rₓ₂) pair, at least one of Aₓ₁ and Aₓ₂ comprises a reactive function suitable for binding of a building block of an organic molecule, preferably a COOH, a NH₂ or an OH group;
   L₂ is a chemical group not comprising any OH, COOH nor NH₂ group, preferably not comprising any reactive function suitable for binding of a building block of an organic molecule;
   n is an integer ranging from 1 to 50, preferably from 2 to 5;
   p is an integer ranging from 1 to n;
   L₃ is a spacer comprising at least 2, preferably from 2 to 18, more preferably from 2 to 10 consecutive atoms between both nitrogen atoms covalently linked to both extremities of L₃, and
(b) implementing the synthesis of the organic molecule on the provided polymer of formula (I) as soluble support.

The polymer of formula (I) presents a functionality of 2 or more, as several building blocks or organic molecules may be bound to the same polymer molecule. Selection of suitable number of consecutive atoms between the nitrogen atoms bonded to L₃ and/or suitable A_{xy} groups allows optimization of the structural features of the polymer of formula (I) depending on the organic molecules to be synthesized and the building blocks to be bound to the support. In particular, the number of at least 2 consecutive atoms in L₃ affords sufficient space between two consecutive organic molecules to be synthesized to be compatible with steric hindrance of said organic molecules. Further, the number of consecutive atoms in L₃ being not more than 18 affords a sufficient density of organic molecules on the support for the organic molecules to behave like in absence of support, and to favor for instance implementation of natural processes such as action of enzymes. The terminating group L₁ is any monovalent group comprising from 1 to 20 atoms, preferably from 1 to 10 atoms. In an embodiment, L₁ is selected from the group consisting of a OH group, a SH group and a NH₂ group. In some embodiments where the polymer of formula (I) is synthesized by solid phase synthesis, the terminating group L₁ is obtained when the polymer is cleaved from the solid support, for example from the solid support resin. In such an embodiment, L₁ may be a COOH-(CH₂)₄-NH- group.

Each R_{xy}, with x ranging from 0 to n, and y being 1 or 2, is independently a H atom or a -(CH₂)₂-A_{xy} group. Independently for each x, at least one of Rₓ₁ and Rₓ₂ is a -(CH₂)₂-A_{xy} group. In some embodiments, independently for each x, only one of Rₓ₁ and Rₓ₂ is a - (CH₂)₂-A_{xy} group and the other of Rₓ₁ and Rₓ₂ is a H atom. In some embodiments, independently for each x, both Rₓ₁ and Rₓ₂ are a -(CH₂)₂-A_{xy} group.

Each A_{xy}, with y being 1 or 2, is independently a chemical group obtainable by the reaction of an organic compound with a double bond. Each A_{xy} results from the reaction of an organic compound comprising a reactive function suitable for reacting with a double bond with a double bond of a polymer of formula (II) as defined below. More precisely, the reaction of the organic compound with a double bond CH=CH of the polymer of formula (II) forms the CH₂-CH₂-A_{xy} moiety. The reactive function suitable for reacting with a double bond may be for instance a double bond, that may react through a metathesis reaction with another double bond, a thiol function (SH), that may react through a thiol-ene reaction with a double bond, an amine function (NH), that may react through a hydroamination reaction with a double bond, or any chemical function suitable for reaction through a Michael addition with a double bond. In some embodiments, the reactive function suitable for reacting with a double bond is thus selected from the group consisting of a double bond, a thiol function and an amine function, preferably a thiol function.

In each (Aₓ₁, Aₓ₂) pair, x ranging from 0 to n, at least one of Aₓ₁ and Aₓ₂ comprises a reactive function suitable for binding of a building block of an organic molecule, preferably a NH₂ or an OH group. In one embodiment, for each (Aₓ₁, Aₓ₂) pair, independently from any other (Aₓ₁, Aₓ₂) pair, only one of Aₓ₁ and Aₓ₂ comprises a reactive function suitable for binding of a building block of an organic molecule. In another embodiment, for each (Aₓ₁, Aₓ₂) pair, independently from any other (Aₓ₁, Aₓ₂) pair, both Aₓ₁ and Aₓ₂ comprise a reactive function suitable for binding of a building block of an organic molecule. The reactive function suitable for binding of a building block of an organic molecule of Aₓ₁ may be the same or different from the reactive function suitable for binding of a building block of an organic molecule of Aₓ₂.

In an embodiment, in all (Aₓ₁, Aₓ₂) pairs, x ranging from 0 to n, only one of Aₓ₁ and Aₓ₂ comprises a reactive function suitable for binding of a building block of an organic molecule. In another embodiment, in all (Aₓ₁, Aₓ₂) pairs, x ranging from 0 to n, both Aₓ₁ and Aₓ₂ comprise a reactive function suitable for binding of a building block of an organic molecule.

n is an integer ranging from 1 to 50. In specific embodiments, n may be 1, 2, 3, 4, 5, 6, 7, 8, 9, 10, 15, 20, 25, 30, 35, 40, 45 or 50. In an embodiment, n is comprised between 2 and 5, preferably n is 2 or 5, in particular n is 2.

L₂ is a chemical group not comprising any OH, COOH nor NH₂ group, preferably not comprising any reactive function suitable for binding of a building block of an organic molecule. L₂ may be for instance selected from the group consisting of a hydrogen atom and an alkyl group.

L₃ is a spacer comprising at least 2, preferably from 2 to 18, more preferably from 2 to 10 consecutive atoms between both nitrogen atoms covalently linked to both extremities of L₃. In one embodiment, L₃ is a spacer comprising 2, 3, 4, 5, 6, 7, 8, 9, 10, 11, 12, 13, 14, 15, 16, 17 or 18 consecutive atoms between both nitrogen atoms covalently linked to both extremities of L₃. In a preferred embodiment, L₃ is a spacer comprising from 4 to 6 consecutive atoms between both nitrogen atoms covalently linked to both extremities of L₃. In another preferred embodiment, L₃ is a spacer comprising from 6 to 9 consecutive atoms between both nitrogen atoms covalently linked to both extremities of L₃. In a more preferred embodiment, L₃ is a spacer comprising 4 consecutive atoms between both nitrogen atoms covalently linked to both extremities of L₃. In another more preferred embodiment, L₃ is a spacer comprising 6 consecutive atoms between both nitrogen atoms covalently linked to both extremities of L₃. In some embodiments, L₃ is a linear or cyclic alkyl group, optionally interrupted or terminated by an aryl group. The at least 2, preferably at least 3, at least 4, at least 5 or at least 6, consecutive atoms in L₃ ensure a sufficient distance between two consecutive reactive functions suitable for binding of a building block of an organic molecule in the polymer of formula (I). One skilled in the art is able to define the minimal number of consecutive atoms in L₃ allowing a sufficient distance between two consecutive reactive functions suitable for binding of a building block of an organic molecule in the polymer of formula (I), while providing a sufficiently high binding capacity. In some embodiments, L₃ is a linear alkyl group comprising from 6 to 18 carbon atoms. In some preferred embodiments, L₃ comprises not more than 9 consecutive atoms between both nitrogen atoms covalently linked to both extremities of L₃.

In a specific embodiment, the polymer of formula (I) provided at step (a) of the process according to the invention is a polymer of formula (Ia): 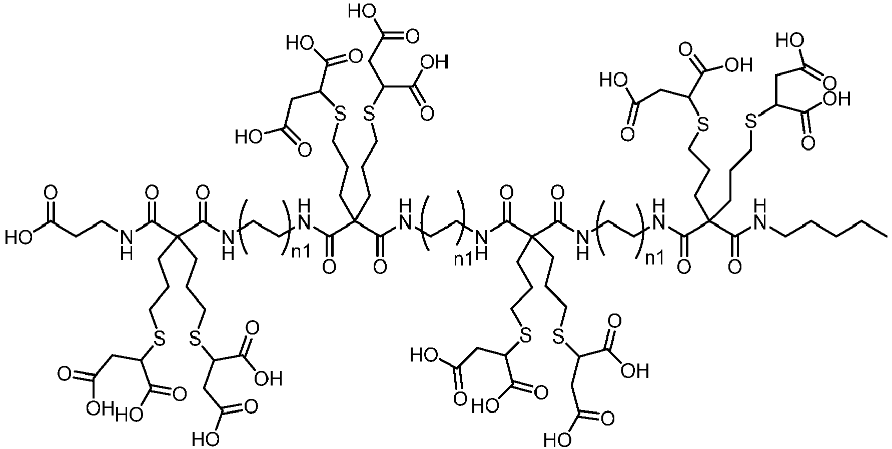 (Ia), wherein n₁ is an integer ranging from 1 to 9, preferably n₁ is 3.

In formula (Ia), L₁ is an OH group; n is 2; L₂ is a n-butyl group and L₃ is a (CH₂-CH₂)ₙ₁ group. The A_{xy} in the polymer of formula (Ia) may be obtained by a thiol-ene reaction on the double bonds of a corresponding intermediate polymer of formula (II).

In a specific embodiment, the polymer of formula (I) provided at step (a) of the process according to the invention is a polymer of formula (Ib):

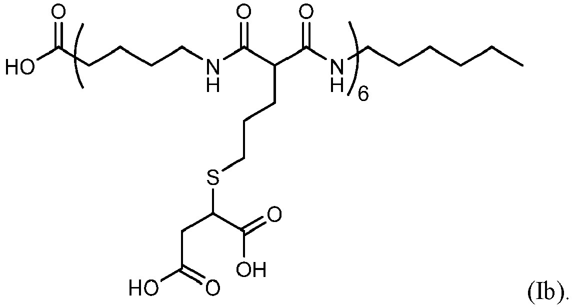

In formula (Ib), L₁ is a COOH-(CH₂)₄-NH- group; n is 5; L₂ is a n-hexyl group and L₃ is a (CH₂-CH₂)₄ group.

The A_{xy} in the polymer of formula (Ib) may be obtained by a thiol-ene reaction on the double bonds of a corresponding intermediate polymer of formula (II).

The implementation of synthesis processes of organic molecules implying soluble supports is well known in the art and one skilled in the art is able to define the suitable conditions and amounts of support and of building blocks depending among others on the organic molecule to be synthesized and the chemical structure of the polymer of formula (I).

The organic molecule to be synthesized may be any organic molecule obtained by repetition of at least two consecutive building blocks. The organic molecule to be synthesized may be for instance selected from the group consisting of a nucleic acid, a peptide, a carbohydrate, or a conjugate thereof. In a preferred embodiment, the organic molecule to be synthesized is a nucleic acid.

In one embodiment, said at least one organic molecule is a biomolecule selected from the group consisting of a nucleotide, an oligonucleotide, a polynucleotide, an amino acid residue, a peptide, a polypeptide, a protein, a monosaccharide, a disaccharide, an oligosaccharide, a polysaccharide.

When the organic molecule is a nucleic acid, the building blocks may be nucleotides, in particular nucleoside triphosphates, or oligonucleotides. Nucleotides may be deoxyribonucleotides or ribonucleotides. Nucleotides may be natural or modified nucleotides bearing functional group on the sugar or the base, for example fluorescent, biotinylated, methylated or aminated nucleotides, in particular fluorescent, biotinylated, methylated or aminated nucleoside triphosphates.

When the organic molecule is a peptide, the building blocks may be amino acids or amino acid oligomers.

The synthesis process may be an enzymatic synthesis process, especially an enzymatic nucleic acid synthesis process.

The structural features of the polymer of formula (I), such as the number of moieties (n) and/or the length of the spacer between two consecutive moieties (L₃) are precisely defined, thus affording a highly reproducible and homogeneous soluble support.

The use of such a polymer of formula (I) in a synthesis process of an organic molecule ensures that the support structure is identical from one synthesis to another synthesis, and thus that the synthesis is always performed in the same conditions and that the same organic molecule is obtained if the same building blocks are brought into the synthesis. Further, as A_{xy}, n and L₃ can be varied, the generic structure of the polymer of formula (I) can be adapted to suit perfectly to the synthesis of any desired molecule, by selecting the appropriate structural features such as A_{xy}, n and L₃. For instance, in order to reduce steric hindrance of the organic molecule to be synthesized, one may select a higher number of consecutive atoms between both nitrogen atoms covalently linked to both extremities of L₃ and/or select A_{xy} such that, for each (Aₓ₁, Aₓ₂) pair, independently from any other (Aₓ₁, Aₓ₂) pair, only one of Aₓ₁ and Aₓ₂ comprises a reactive function suitable for binding of a building block of an organic molecule.

If the polymer of formula (I) has been synthetized from at least one molecule bound to a solid support, then the polymer of formula (I) has to be cleaved from said solid support before step (b) of implementing the synthesis of the organic molecule on the provided polymer of formula (I) as soluble support. The solid support may be a resin. For example, if the polymer of formula (I) has been synthetized from the intermediate polymer of formula (II) bounded to a solid support via L₁, preferably bounded to a resin via L₁ (see below), then the polymer of formula (I) has to be cleaved from said solid support, preferably from said resin, before step (b) of implementing the synthesis of the organic molecule on the provided polymer of formula (I) as soluble support. This cleavage step may be carried out, if necessary, by suitable techniques, in particular in an acid medium, for example using trifluoroacetic acid.

Step (b) of the synthesis process of the invention is a step of implementing the synthesis of the organic molecule on the provided polymer of formula (I) as soluble support. Step (b) may be implemented in any suitable conditions known in the art. For instance, step (b) may be implemented in presence of a suitable enzyme. For the synthesis of nucleic acid, a suitable enzyme can be a Terminal deoxynucleotidyltransferase (TdT), a DNA polymerase, a DNA primase, a RNA polymerase, a reverse transcriptase, a RNA ligase or a DNA ligase.

In some embodiments, step (b) comprises a first sub-step (ba) consisting of binding at least one building block of the organic molecule to be synthesized to at least one of Aoi, A₀₂, Aₚ₁ and Aₚ₂, and a second sub-step (bb) consisting of exposing the at least one bound building block obtained at step (ba) to suitable conditions for implementing the binding of a further building block and, iteratively, the synthesis of the organic molecule.

In an embodiment, in sub-step (ba), building blocks of the organic molecule to be synthesized are bound to only one of Aₓ₁ and Aₓ₂ for each (Aₓ₁, Aₓ₂) pair, independently from any other (Aₓ₁, Aₓ₂) pair, x ranging from 0 to n.

In a preferred embodiment, in sub-step (ba), building blocks of the organic molecule to be synthesized are bound to both Aₓ₁ and Aₓ₂ in all (Aₓ₁, Aₓ₂) pairs, x ranging from 0 to n.

In an embodiment, sub-step (bb) comprises contacting the at least one bound building block obtained at step (ba) with at least one equivalent of another building block of the organic molecule to be synthesized.

Step (bb) may be implemented consecutively several times depending on the number of building blocks to be bound.

The process for the synthesis of an organic molecule according to the invention may comprise further steps, for instance classical steps known in the art.

The process for the synthesis of an organic molecule according to the invention may further comprise at least one step (c) consisting of separating the obtained organic molecule, preferably still bound to the soluble support, from the monomeric building blocks and other small molecular reagents and byproducts. Said step (c) may be implemented by any suitable technique known in the art, for instance by precipitation, extraction, chromatography and/or nanofiltration. The presence of the polymer of formula (I) as soluble support thus allows the washing in particular by filtration.

The process for the synthesis of an organic molecule according to the invention may further comprise at least one step (d) consisting of separating the obtained organic molecule from the soluble support. Said step (d) may be implemented by any suitable technique known in the art. For instance, following the synthesis of a nucleic acid, step (d) may be implemented by using Uracile Specific Excision Reagent (USER^{™}) composed by a Uracile DNA glycosylase enzyme, or by using nicking endonucleases, or by alkaline hydrolysis in presence of sodium hydroxide (NaOH), or by photocleavage, (phosphothiolate...). After such specific enzymatic cleavage, the soluble support is then reusable for subsequent synthesis of organic molecules according to step (b).

### Use in molecular biology and biochemistry assays

This invention further relates to the use of a polymer of formula (I) as a soluble support in molecular biology and biochemistry assays.

Examples of molecular biology and biochemistry assays in which the polymer of formula (I) according to the present invention is useful include, but are not limited to, ligand binding assays, immunoassays, enzyme assays, nucleic acid hybridization assays, affinity purification, and the like.

### Polymer

Another object of the invention is a polymer of formula (I) as defined above. All embodiments relating to the polymer features and disclosed for the process for the synthesis of organic molecules apply similarly to the polymer of formula (I) by itself.

In some embodiments, in particular when the polymer of formula (I) is synthesized by solid phase synthesis implying a resin, the polymer of formula (I) is covalently bound to a resin through its L₁ terminating group.

The polymer of formula (I) according to the invention may be synthesized by any suitable method known in the art. A particularly advantageous synthesis process of the polymer of formula (I) described below is another object of the invention.

### Polymer synthesis process

Another object of the invention is a process for the synthesis of a polymer of formula (I) as defined above, wherein the process comprises the steps of:
a1) Providing an intermediate polymer of formula (II) 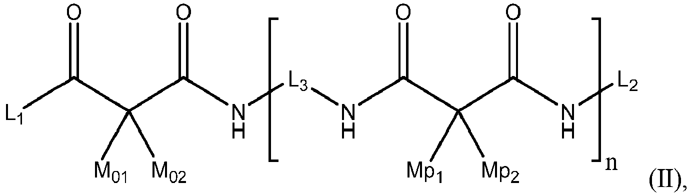 wherein:
   each M_{wz}, with w ranging from 0 to n, and z being 1 or 2, is independently a H atom or an allyl -CH₂-CH=CH₂ group; provided that, in each (M_{w1}, M_{w2}) pair, at least one of M_{w1} and M_{w2} is an allyl -CH₂-CH=CH₂ group, preferably in each (M_{w1}, M_{w2}) pair, both M_{w1} and M_{w2} are allyl CH₂-CH=CH₂ groups; and
   L₁, L₂, L₃, n and p are as defined for the formula (I) above;
      and
b1) Reacting the intermediate polymer of formula (II) provided at step (a1) with an organic compound A, wherein compound A comprises:
   - a reactive function suitable for reacting with a double bond, preferably a reactive function selected from the group consisting of a double bond, a thiol function (SH) and an amine function (NH₂), and
   - a reactive function suitable for binding of a building block of an organic molecule, preferably a COOH, a NH₂ or an OH group,
      in conditions suitable for implementing a reaction between at least one double bond of the allyl moieties of the intermediate polymer of formula (II) and the reactive function of compound A suitable for reacting with a double bond.

The intermediate polymer of formula (II) provided at step (a1) may be synthesized by any suitable method known in the art.

In a particular embodiment, the intermediate polymer of formula (II) is selected from the group consisting of:

| | |
|---|---|
| (IIa) | 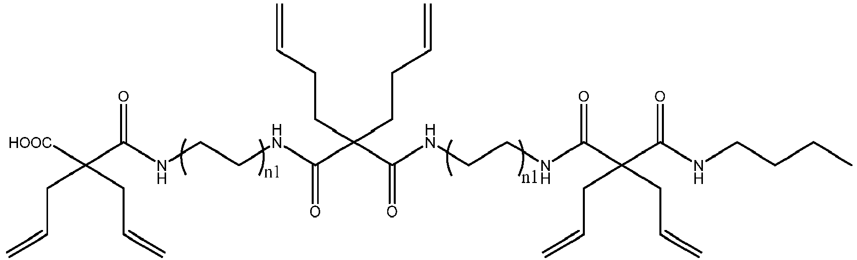 |
| (IIb) | 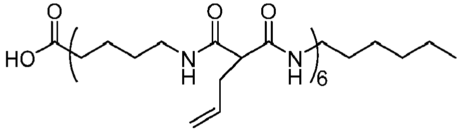 |
| (IIc) | 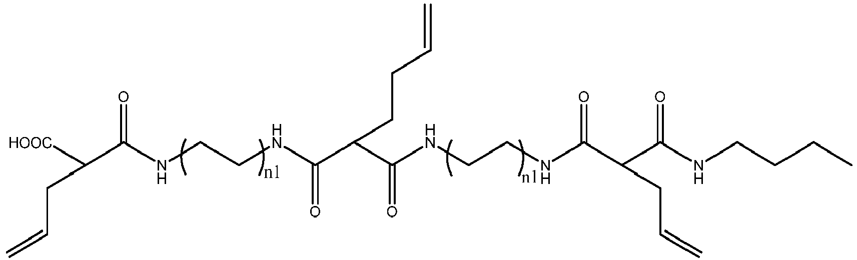 |
| (IId) | 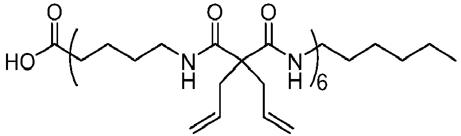 |

wherein each n1 is as defined for the formula (Ia) above.

Step (b1) of the process of the invention may be implemented by any suitable method known in the art. Compound A may be any organic compound comprising at least one thiol SH reactive function or at least one double bond or at least one amine NH₂ reactive function, and a reactive function suitable for the binding of a building block of an organic molecule. In one embodiment, compound A is an organic compound comprising at least one thiol SH reactive function and a reactive function suitable for the binding of a building block of an organic molecule. In a preferred embodiment, compound A is mercaptosuccinic acid.

In some embodiments, the reactive function suitable for the binding of a building block of an organic molecule of compound A is protected by a protecting group in order not to be affected by the implementation of the reaction between the reactive function suitable for reacting with a double bond and at least one double bond of the allyl moieties of the intermediate Polymer of formula (II). Suitable protecting groups are part of current knowledge in the art.

When the reactive function suitable for the binding of a building block of an organic molecule of compound A is a OH group, it may be protected by any suitable protecting group known in the art and that cannot be deprotected in the reactive conditions used for implementing the thiol-ene reaction. For instance, the OH group may be protected as an acetal, a benzylic ether or a silyl ether.

When the reactive function suitable for the binding of a building block of an organic molecule of compound A is a NH₂ group, it may be protected by any suitable protecting group known in the art and that cannot be deprotected in the reactive conditions used for implementing the thiol-ene reaction. For instance, the NH₂ group may be protected as an imide, an amide, a carbamate, such as tert-butyloxycarbonyl Boc and fluorenylmethoxycarbonyl Fmoc, an imine, an enamine, a sulfonyl derivative, a N-sulfenyl derivative, a N-alkyl derivative or a N-silyl derivative.

The reaction between the reactive function suitable for reacting with a double bond and at least one double bond of the allyl moieties of the intermediate Polymer of formula (II) may be a hydroamination reaction between an amine function (NH) and a double bond, a metathesis reaction between two double bonds, a thiol-ene reaction between a thiol function (SH) and a double bond or a Michael addition between a double bond and any chemical function suitable for reaction through a Michael addition with a double bond. The thiol-ene reaction, also known as alkene hydrothiolation, is an organic reaction between a thiol and an alkene to form a thioether, and is known as a "click-chemistry" reaction. Typical conditions for implementing a thiol-ene reaction involve either free-radical addition conditions, using for instance light, heat or a radical as initiator, or Michael addition conditions, in presence of a base or acid catalyst.

In a particular embodiment, step (b1) is carried out in at least one, preferably all, conditions below:
- in dimethylformamide (DMF),
- at 60°C,
- during about 3 hours, and
- is initiated by azobisisobutyronitrile (AIBN).

In one embodiment, the intermediate polymer of formula (II) is bound to a solid support via L₁, preferably the solid support is a resin. According to this embodiment, the process for the synthesis of a polymer of formula (I) may comprise an additional step (c1) of cleaving between the solid support and L₁, in particular between the resin and L₁. The steps may be performed in the order (b1), then (c1) or (c1), then (b1). Preferably, step (b1) is performed before step (c1). Step (c1) may be carried out in any suitable conditions, for instance in a trifluoroacetic acid solution, at room temperature and/or during about 2 hours.

The synthesis process of the invention may comprise a further step (d1) of reacting the polymer obtained at step (b1) with one or more suitable deprotecting agent when at least one reactive function of the polymer obtained at step (b1) is protected with a protecting agent.

### Intermediate polymer

Another object of the invention is an intermediate polymer of formula (II) as defined above.

In a specific embodiment, the intermediate polymer of formula (II) is the compound of formula (IIe) or (IIf): 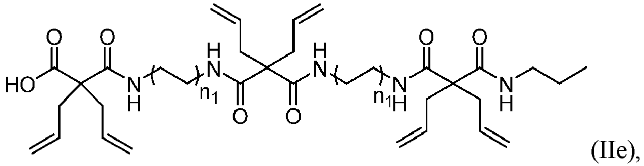 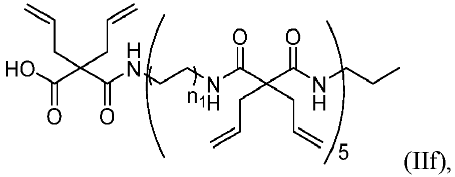 wherein each n₁ is an integer ranging from 1 to 9, preferably n₁ is 3.

The polymer of formula (II) may be synthesized by any suitable polymerization technique known in the art for forming such polymer. For instance, the polymer of formula (II) may be synthesized in solid phase by amidation techniques: reaction between amine and ester or amine and carboxylic acid catalysed by a carbodiimide such as EDC (1-éthyl-3-(3-dimethylaminopropyl)carbodiimide) or DIC (N,N'-diisopropylcarbodiimide). The double bonds of the monomers may be modified either by a click chemistry method, thiolene or by hydroamination.

In some embodiments, in particular when the polymer of formula (II) is synthesized by solid phase synthesis implying a resin, the polymer of formula (II) is covalently bound to a resin through its L₁ terminating group.

In the present specification, any interval or range of values should be understood as including the limits thereof, unless otherwise specified.

In the present specification, the term "comprise" and any variation thereof are open terms, meaning that when an object or a process comprises a component or a step, it does not exclude the presence of further components or steps. In some embodiments, the term "comprise" and any variation thereof may be construed as meaning "essentially consist of' or even "consist of'.

### EXAMPLES

The present invention is further illustrated by the following examples, which are illustrative, and not limitative, of the present invention.

### Example 1: Synthesis of an intermediate polymer of formula (II)

### 1. Activation of a Wang resin

### 1.a. Esterification reaction

A Wang resin was placed in dichloromethane for 30 minutes. Then, Fmoc-5-AvaOH and N,N'-diisopropylcarbodiimide (DIC) were added in the dichloromethane containing the resin for 24 hours and 30 minutes, first at 0°C for 30 min and then at room temperature for 24 hours, with the addition of 4-dimethylaminopyridine (DMAP). The resin was then filtered through a solid phase extraction tube (SPE tube) and washed several times with dichloromethane and diethyl ether.

### 1.b. Acetylation of unreacted OH

To avoid a secondary reaction of the unreacted alcohol moieties present on the resin, acetic anhydride was added for 2 hours at room temperature in the dichloromethane solution in the presence of N,N-diisopropylethylamine (DIPEA). This step was followed by a series of dichloromethane/diethyl ether washes.

### 1.c. De-protection of Fmoc-5-ava-OH

In order to have a free amine, Fmoc was removed using a solution of 4-Methylpiperidine in DMF for 2x20 minutes at room temperature. At the end the resin was rinsed with dichloromethane followed by a wash with diethyl ether.

### 2. Polymerization reaction

2.a. The activated resin was reacted with the monomer, diethyl diallylmalonate, optionally activated as diphenyl diallylmalonate, in DMF. This reaction was catalyzed by triazabicyclodecene (TBD) at 60°C for 4 hours. At the end of the reaction, the resin underwent a series of washes with dichloromethane and diethyl ether.
2.b. The previously washed resin was reacted with pentane diamine. This reaction was carried out in the presence of triazabicyclodecene (TBD) at 60°C for 4 hours. A washing step was then carried out as in step 2.a.

Steps 2.a. and 2.b. were repeated 3 times to obtain an oligomer with 3 units. Thus, two oligomers with 3 units were synthesized with a perfect control of their length and the terminal functions. One oligomer comprises an NH₂ as terminal function, and the other comprises an alkyl group as terminal function.

### 3. Cleavage step

This step consists in the cleavage of the resin and the oligomer comprising an alkyl group as terminating function with 3 units in acid medium. This step lasted 2 hours and was carried out at room temperature under stirring. The recovered product was dried under vacuum.

### 4. Coupling of two oligomers

The oligomer with an alkyl group terminating function (L₂), which was separated from the resin in step 3. comprises a COOH function at the end of the chain (Li). It was dissolved in dimethylformamide (DMF) and reacted with another oligomer having 3 units and an NH₂ terminating function and still bound to the resin surface. Step 4. lasted 3 hours and was carried out at room temperature in the presence of DIC. The resulting oligomer has 6 units and was bound to the resin.

The 6 units oligomer may be cleaved from the resin with cleavage conditions similar to those of cleavage step 3 in order to obtain an unbound polymer of formula (II) according to the invention.

### Example 2: Synthesis of a polymer of formula (I)

After step 4. of Example 1, the following steps 5. and 6. were carried out:

### Step 5: Thiolene step (click chemistry)

This is the reaction of mercaptosuccinic acid through its -SH on the double bonds of the 6 units of the previously synthesized oligomer which has 6 units and was bound to a resin. This reaction was initiated by AIBN at 60°C in DMF for 3 hours. At the end of the reaction, the substance was recovered in a SPE tube and washed several times with DMF and then with diethyl ether.

### Step 6: Cleavage of the resin and 6 units oligomer:

The obtained 6 units polymer on which mercaptosuccinic acid is bonded was cleaved from the resin with cleavage conditions similar to those of cleavage step 3 above, in order to obtain an unbound polymer of formula (I) according to the invention.

### Example 3: Controlled-Enzymatic and template-independent ssDNA oligonucleotide synthesis using a soluble support

The polymer of formula (I) produced in Example 2 was used as a soluble support in a process of enzymatic nucleic acid synthesis, the soluble support replacing the commonly used solid support. A 33-mer oligonucleotide serves as a synthesis initiator and was first coupled to the soluble support before adding the nucleic acid building blocks. The process was carried out using 3' -*O*-NH₂-dNTPs as organic molecule building block and a terminal deoxynucleotidyl transferase (TdT) as reacting enzyme. The protecting 3'-*O*-NH₂ group was removed using 700 mM sodium nitrite (NaNO₂) in 1 M sodium acetate buffered at pH 4.5-5.0 with acetic acid.

The process was carried out as known in the art, including steps of:
(a) contacting the functionalized soluble support with 3 '-*O*-NH₂-dNTPs in presence of TdT, in conditions suitable to couple the 3'-*O*-NH₂-dNTPs to the 3' terminus of the 33-mer oligonucleotide, thereby obtaining a 3' protected 34-mer oligonucleotide;
(b) washing the excess of 3'-*O*-NH₂-dNTPs and other reagents, *e.g*., by vacuum filtration;
(c) contacting the soluble support with a deprotecting reagent in conditions suitable to remove the protecting group from the 3' protected 34-mer oligonucleotide,
(d) optionally, repeating (b) and (c),
(e) repeating steps (a) to (d) until the desired oligonucleotide is obtained.

### Example 4: PCR using a T7 terminator primer immobilized on a polymer-based soluble support for large-scale mRNA production.

The synthesis of messenger RNA molecules on a large scale represents a major industrial challenge mainly due to the low production yields of conventional methods, as well as the intrinsic instability of these molecules at room temperature. In fact, to produce messenger RNA molecules, conventional methods require a first step of grafting the template gene of interest onto a functionalized chromatographic resin or of cloning the template gene in a plasmid vector, in the form of double-stranded DNA; followed by an in vitro transcription step. This second step, which is carried out in bioreactors, requires the use of synthetic enzymes or enzymatic complexes, such as the RNA polymerase of phage T7, but also of maturation enzymes specialized in the addition of a cap at the 5' end and of the polyadenylation at the 3' end. Finally, the produced messenger RNA molecules must be purified by chromatography in order to be separated from synthetic enzymes and template DNA. However, although the synthesis processes are mastered at the laboratory scale, none of these steps is optimized for industrialization and mass production, the reaction yields being directly linked to the quantity of starting material and dependent from one stage to another. Moreover, in addition to the problems associated with the successive use of several enzymes, it should be noted that RNA polymerases produce a large number of abortive strands that very often result in the synthesis of truncated RNA strands. The polymer of the invention can overcome these issues by increasing the quantity of starting template DNA.

### RNA polymerization

In a first step, one or more copies, in a single-stranded DNA form, of the complementary strand of a gene to be transcribed, are coupled to the polymer-based soluble support of the invention. The full length of this complementary strand can be coupled to the polymer-based soluble support; or alternatively, only a complementary fragment of the gene to be transcribed is coupled to the polymer-based soluble support, serving as a primer for a first step of amplification in presence of the template gene in ssDNA form, thereby yielding the full complementary strand of the gene being coupled to the polymer-based soluble support.

In a second step, synthesis cycles can be carried out, comprising three successive steps and repeated between 1 and 50 times:
a- Denaturation at 95°C of the complementary strand attached to the polymer-based soluble support, in the presence of an RNA primer specific to the 3' end of this complementary strand and of a thermostable RNA polymerase (e.g., the mutated Tgo DNA polymerase from *Thermococcus gorgonarius* described by Cozens et al., 2012. 10 Proc Natl Acad Sci USA. 109(21):8067-72). Denaturation can take place in a bioreactor capable of stirring, controlling the temperature and filtering, or directly in a thermal cycler;
b- Hybridization of the RNA primer on the complementary strand at a temperature ranging from about 50°C to about 70°C;
c- Synthesis of the messenger RNA strand, complementary to the template ssDNA strand, by the polymerase, at temperatures ranging from about 60°C to about 75°C.

In a third step, successive ultrafiltrations or filtrations of the reaction medium are carried out with a buffer or water, in order to remove salts, nucleotides and the polymerase. This washing step allows to retains only the RNA strands and the template strand attached to the soluble support. Filtration can take place in a bioreactor capable of stirring, controlling the temperature and filtering, or directly in ultrafiltration units. Finally, the sample can be incubated in the presence of an enzyme mixture able to perform a 5' capping and a 3' poly-adenylation to the newly produced messenger RNA. Before additional rounds of washing and optionally, of DNAse/protease treatment, and ultimately, recovery of the messenger RNA strands.

### "One-pot" RNA synthesis

In a first step, one or more copies, in a single-stranded DNA form, of the complementary strand of a gene to be transcribed, are coupled to the polymer-based soluble support. The attached strand should have a plurality of deoxythymidine at their 5' end, preferably contiguous, preferably between 200 and 300. Coupling to the soluble support of the invention can be made via the 5' end, the 3' end or both indiscriminately. As previously explained, the full length of this complementary strand can be coupled to the polymeric scaffold; or alternatively, only a complementary fragment of the gene to be transcribed is coupled to the polymeric scaffold (with its plurality of deoxythymidine at the 5' end), serving as a primer for a first step of amplification in presence of the template gene in ssDNA form, thereby yielding the full complementary strand of the gene being coupled to the polymer-based soluble support. In a second step, synthesis cycles can be carried out, comprising three successive steps and repeated between 1 and 50 times:
a- denaturation at 95°C of the complementary strand attached to the polymer-based soluble support, 15 in the presence of a 5' capped RNA primer specific to the 3' end of this complementary strand and of a thermostable RNA polymerase (e.g., the mutated Tgo DNA polymerase from Thermococcus gorgonarius described by Cozens et al., 2012. Proc Natl Acad Sci USA. 109(21):8067-72). Denaturation takes place in a bioreactor capable of stirring, controlling the temperature and filtering, 20 or directly in a thermal cycler;
b- hybridization of the RNA primer on the complementary strand at a temperature ranging from about 50°C to about 70°C;
c- synthesis of the messenger RNA strand, complementary to the template ssDNA strand, by the polymerase, at temperatures ranging from about 60°C to about 75°C.

In a third step, successive ultrafiltrations or filtrations of the reaction medium are carried out with a buffer or water, in order to remove salts, nucleotides and the polymerase. This washing step allows to retains only the RNA strands and the template strand attached to the soluble support. Filtration can take place in a bioreactor capable of stirring, controlling the temperature and filtering, or directly in ultrafiltration units.

Finally, the sample can optionally be incubated in the presence of DNAse/proteases, before additional rounds of washing and ultimately, recovery of the messenger RNA strands.

## Claims

1. A process for the synthesis of an organic molecule, comprising the steps of:
(a) Providing a polymer of formula (I): 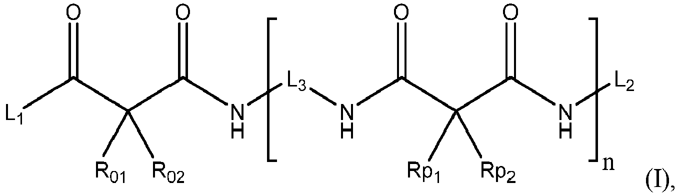 wherein
L₁ is a terminating group, preferably selected from the group consisting of a OH group, a SH group and a NH₂ group;
each R_{xy}, with x ranging from 0 to n, and y being 1 or 2, is independently a H atom or a - (CH₂)₂-A_{xy} group, wherein, independently for each x,
at least one of Rₓ₁ and Rₓ₂ is a -(CH₂)₂-A_{xy} group,
each A_{xy} is independently a chemical group obtainable by the reaction of an organic compound with a double bond, preferably via a thiol-ene reaction, and in each (Rₓ₁, Rₓ₂) pair, at least one of Aₓ₁ and Aₓ₂ comprises a reactive function suitable for binding of a building block of an organic molecule, preferably a COOH, a NH₂ or an OH group;
L₂ is a chemical group not comprising any OH, COOH nor NH₂ group, preferably not comprising any reactive function suitable for binding of a building block of an organic molecule;
n is an integer ranging from 1 to 50, preferably from 2 to 5;
p is an integer ranging from 1 to n;
L₃ is a spacer comprising at least 2 consecutive atoms between both nitrogen atoms covalently linked to both extremities of L₃,
and
(b) Implementing the synthesis of the organic molecule on the polymer of formula (I) provided at step (a) as soluble support.

2. The process according to claim **1,** wherein the organic molecule is a nucleic acid, a nucleotide, an oligonucleotide, a polynucleotide, an amino acid residue, a peptide, a polypeptide, a protein, a monosaccharide, a disaccharide, an oligosaccharide, a polysaccharide, or a conjugate thereof.

3. The process according to claim **2,** wherein the process is an enzymatic nucleic acid synthesis process.

4. The process according to claim **1** or **2,** wherein L₁ is selected from the group consisting of a OH group, a SH group and a NH₂ group, preferably L₁ is a -OH group.

5. The process according to anyone of claims **1** to **4,** wherein, in each (Aₓ₁, Aₓ₂) pair, both Aₓ₁ and Aₓ₂ comprise a reactive function suitable for binding of a building block of an organic molecule, preferably a COOH, a NH₂ or an OH group.

6. The process according to anyone of claims **1** to **5,** wherein L₃ comprises 6 consecutive atoms between both nitrogen atoms covalently linked to both extremities of L3.

7. The process according to anyone of claims **1** to **6,** wherein n is comprised between 2 and 5, preferably n is 2 or 5, more preferably n is 2.

8. The process according to anyone of claims **1** to **7,** wherein L₂ is a hydrogen atom or an alkyl group.

9. The process according to anyone of claims **1** to **8,** wherein the soluble support of formula (I) is a polymer of formula (Ia): 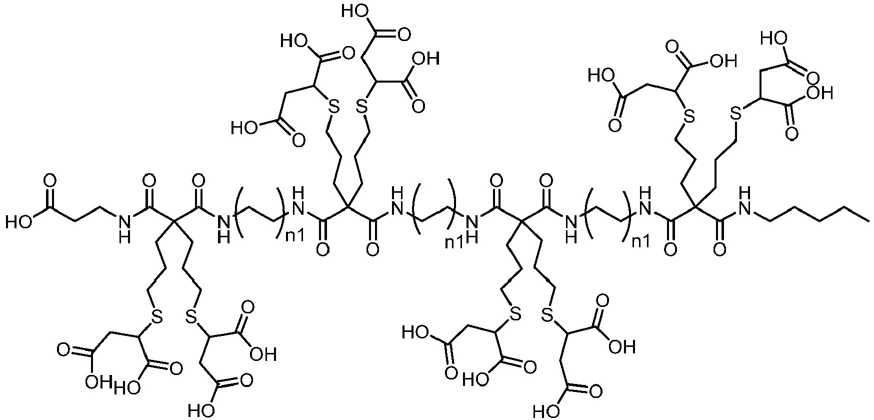 wherein n₁ is an integer ranging from 1 to 9, preferably n₁ is 3.

10. The process according to anyone of claims 1 to 9, further comprising at least one step (c) consisting of separating the obtained organic molecule, preferably still bound to the soluble support, from the monomeric building blocks and other small molecular reagents and byproducts.

11. The process according to anyone of claims **1** to **10,** further comprising at least one step (d) consisting of separating the obtained organic molecule from the soluble support.

12. A polymer of formula (I) 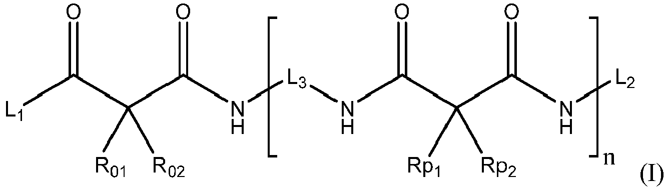 wherein L₁, L₂, L₃, R_{xy}, n and p are as defined in anyone of claims **1** to **9.**

13. Use of a polymer according to claim **12,** as a soluble support.

14. Use of a polymer according to claim **12,** as a soluble support in synthesis process of nucleic acids, preferably a liquid-phase synthesis process of nucleic acids.

15. The use according to claim **14,** wherein the synthesis process of nucleic acids is a *de novo* nucleic acid synthesis process, a template-dependent nucleic acid synthesis process or a nucleic acid assembly process.

16. The use according to claim **13,** as a soluble support in molecular biology and biochemistry assays.

17. The use according to claim **16,** wherein the molecular biology and biochemistry assay is a nucleic acid hybridization assay, a PCR, or a DNA template amplification prior to in vitro transcription.

18. A process for the synthesis of a polymer of formula (I) as defined in anyone of claims **1** to **9,** wherein the process comprises the steps of:
a1) Providing an intermediate polymer of formula (II) 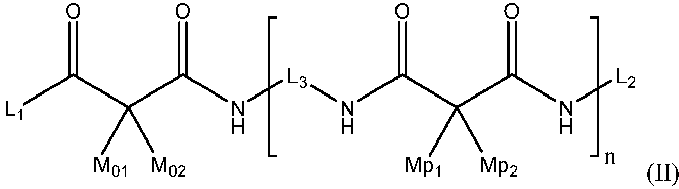 wherein
each M_{wz}, with w ranging from 0 to n, and z being 1 or 2, is independently a H atom or an allyl -CH₂-CH=CH₂ group; provided that, in each (M_{w1}, M_{w2}) pair, at least one of M_{w1} and M_{w2} is an allyl -CH₂-CH=CH₂ group, preferably in each (M_{w1}, M_{w2}) pair, both M_{w1} and M_{w2} are allyl CH₂-CH=CH₂ groups; and
L₁, L₂, L₃, n and p are as defined as defined in anyone of claims **1** to **9;** and
b1) Reacting the intermediate polymer of formula (II) provided at step (a1) with a compound A, wherein compound A comprises:
- a reactive function suitable for reacting with a double bond, preferably a reactive function selected from the group consisting of a double bond, a thiol function (SH) and an amine function (NH₂), and
- a reactive function suitable for fixation of a building block of an organic molecule, preferably a COOH, a NH₂ or an OH group,
in conditions suitable for implementing a reaction between at least one double bond of the allyl moieties of the intermediate polymer of formula (II) and the reactive function of compound A suitable for reacting with a double bond.

19. An intermediate polymer of formula (II): 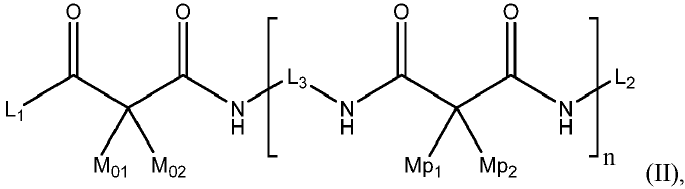 wherein
M_{wz}, L₁, L₂, L₃, n and p are as defined in claim **18.**

20. The intermediate polymer according to claim **19,** wherein said intermediate polymer is selected from the group consisting of: 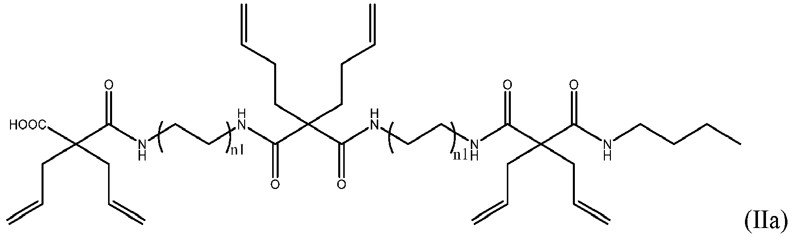 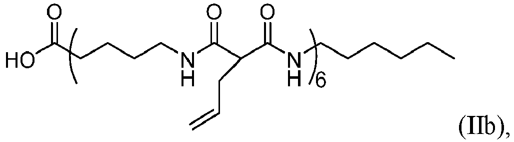 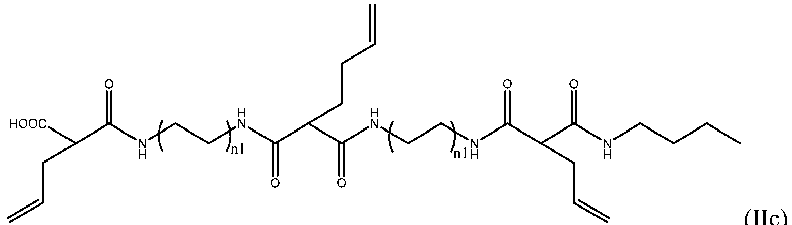 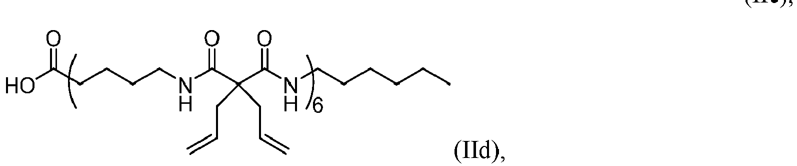 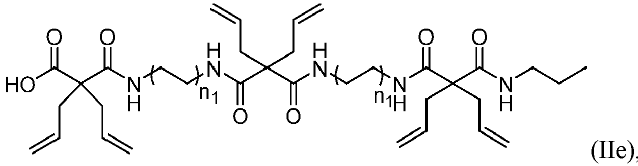 and 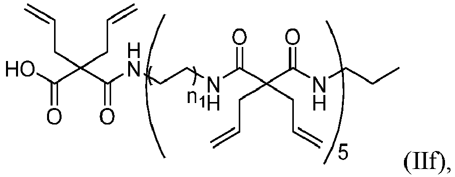 wherein each n₁ is an integer ranging from 1 to 9, preferably n₁ is 3.
